# EUROPEAN PATENT APPLICATION

(11) **EP 4 101 463 A1**
(43) Date of publication of application: **14.12.2022**
(21) Application number: 21747850.2
(22) Date of filing: 01.02.2021
(51) Int. Cl.: A61K 39/395, A61K 48/00, A61P 13/12, A61P 37/06, C07K 16/28, C12N 15/113, C12Q 1/66, G01N 33/53, A61K 31/7088, A61K 31/7105, A61K 31/713

(54) **METHOD FOR ASSESSING POSSIBILITY OF ONSET OR PROGRESSION OF CHRONIC KIDNEY GRAFT REJECTION AND CHRONIC KIDNEY DISEASE, TEST KIT, AND PHARMACEUTICAL COMPOSITION**

(30) Priority: 31.01.2020 JP 2020015303
(71) Applicant: National University Corporation Hokkaido University, Sapporo-shi, Hokkaido 060-0808 (JP)
(72) Inventor: MURAKAMI Masaaki, Sapporo-shi, Hokkaido 060-0808 (JP); KAMIMURA Daisuke, Sapporo-shi, Hokkaido 060-0808 (JP); TAKADA Yusuke, Sapporo-shi, Hokkaido 060-0808 (JP)
(74) Representative: Wasner, Marita
(86) International application number: PCT/JP2021/003576
(87) International publication number: WO 2021/153798

(57) **Abstract**

The present invention provides a method for assessing the possibility of onset or progression of chronic kidney graft rejection and chronic kidney disease in a subject, by using, as an indicator, the mass of the SYT17 protein in a urine sample collected from the subject. The present invention also provides: a pharmaceutical composition that is for prophylaxis and/or treatment of chronic kidney graft rejection or chronic kidney disease and that contains at least one substance selected from the group consisting of specific antibodies for SYT17 and derivatives thereof, and inhibitory nucleic acids against SYT17; and a test kit that is for assessing the possibility of onset or progression of chronic kidney graft rejection and chronic kidney disease and that contains a specific antibody for SYT17 or a derivative thereof.

## Description

### FIELD

The present invention relates to a method for assessing the possibility of onset or progression of chronic kidney allograft rejection and chronic kidney disease, a test kit for the assessment, and a pharmaceutical composition for preventing and/or treating chronic kidney allograft rejection and chronic kidney disease.

### BACKGROUND

Chronic kidney disease (CKD) is a condition in which either or both of renal disorder, typified by proteinuria, and renal hypofunction, as indicated by glomerular filtration rate persist for 3 months or longer. One of eight adults suffer from chronic kidney disease in Japan. When chronic kidney disease progresses to end-stage renal failure, the effect of drug treatment cannot be expected, and therefore many patients unavoidably undergo dialysis. Since dialysis is a symptomatic therapy, a patient having end-stage renal failure needs to continue undergoing dialysis throughout his/her lifetime, which places physically and economically significant burdens on the patient. Furthermore, the increasing cost of dialysis has become an issue for the medical economy.

In the diagnosis of chronic kidney disease, renal function is assessed by biomarkers such as urinary protein, urinary albumin, serum creatinine (Cre), blood urea nitrogen (BUN), and estimated glomerular filtration rate (eGFR). Although these biomarkers have the advantage of being easily measured and calculated, the biomarkers are not suitable for early detecting the progression of renal dysfunction with sufficient sensitivity.

Kidney transplantation is the only curative therapy for end-stage renal failure. Data from the United State Renal Data System in 2013 (Non Patent Literature 1) indicate that survival rate of patients having undergone kidney transplantation is better than that of dialysis patients.

Allograft immunorejection strongly influences the success rate for kidney transplantation. Recent developments in immunosuppressive drugs and medical technologies have markedly reduced the risk of acute immunorejection. However, chronic rejection, which progresses comparatively slowly and is typified by chronic active antibody-mediated rejection (CAAMR), remains difficult to overcome.

Chronic rejection may occur 3 months or longer after kidney transplantation. In many cases, chronic rejection gradually progresses without any clear clinical symptom observed, and hence, appropriate treatment is not given, which highly possibly results in loss of renal graft function. Typically, chronic rejection is diagnosed by biopsy of a graft. However, biopsy is burdensome for a subject tested and is not a simple procedure. Furthermore, biopsy sometimes causes underestimation of rejection grade and oversight of a lesion. Therefore, a simple and accurate method for determining the possibility of onset or progression of chronic rejection is useful for diagnosis of chronic rejection, determination of appropriate timing of biopsy, and implementation of prophylactic or therapeutic intervention for chronic kidney disease with appropriate timing, and is of great importance in kidney transplantation therapy.

A family of proteins called synaptotagmin is known. Synaptotagmin is a membrane protein present on synaptic vesicles and has been identified as calcium-phospholipid binding molecule. Synaptotagmin is considered to function as a calcium sensor in exocytosis from synaptic vesicles due to its ability to bind calcium ions.

There are 17 isoforms of the synaptotagmin family reported to exist in humans, and many of the isoforms have a transmembrane domain on the N-terminal side and two C2-domains in a cytoplasmic domain on the C-terminal side. A protein called Synaptotagmin-17 (SYT17) has two C2-like domains structurally homologous with the C2-domains on the C-terminal side that is common in a human synaptotagmin family, but does not have a transmembrane domain on the N-terminal side.

It has been reported that SYT17 protein is expressed in the brain and kidneys, that SYT17 protein is particularly increased in the hippocampus of a kainic acid-induced rat epileptic seizure model and in the proximal tubules in the kidney after ischemia-reperfusion injury, and that the expression level of SYT17 varies depending on the disease state (Non Patent Literature 2 and Non Patent Literature 3). However, there have been no reports on the functional role of SYT17 protein and changes in its expression levels in chronic kidney disease and chronic rejection.

### CITATION LIST

### NON PATENT LITERATURES

Non Patent Literature 1: UNITED STATES RENAL DATA SYSTEM, [online], [search on January 28, 2020], the Internet <URL: https://www.usrds.org/>
Non Patent Literature 2: Jang, Y. S., et al., 2004, Brain Res. 999: 203
Non Patent Literature 3: Han, K. H., et al., 2007, Am. J. Physiol. Renal Physiol. 292: F100

### SUMMARY

### TECHNICAL PROBLEM

An object of the present invention is to provide a clinical indicator that can be used in assessing the possibility of onset or progression of chronic kidney allograft rejection or chronic kidney disease and can provide appropriate information for implementing renal graft biopsies and necessary intervention.

### SOLUTION TO PROBLEM

The present inventors found that SYT17 protein was present in higher amounts in urine specimens of patients developing chronic kidney allograft rejection or chronic kidney disease, compared with healthy individuals, and completed the following invention.
(1) A method for assessing the possibility of onset or progression of chronic kidney allograft rejection or chronic kidney disease in a test subject by using the level of SYT17 protein in a urine-derived specimen collected from the test subject as an indicator.
(2) The method according to (1), comprising the steps of: measuring the level of SYT17 protein in the urine-derived specimen collected from the test subject; comparing the level of SYT17 protein in the urine-derived specimen collected from the test subject with a cutoff value, the cutoff value being determined by measuring beforehand the levels of SYT17 protein in urine-derived specimens collected from a plurality of patients developing chronic kidney allograft rejection or chronic kidney disease and a plurality of control subjects, and conducting an ROC analysis using these measured levels; and, when the level of SYT17 protein in the urine-derived specimen collected from the test subject exceeds the cutoff value, determining that the test subject is developing or has a high possibility of developing chronic kidney allograft rejection or chronic kidney disease.
(3) The method according to (1), comprising the steps of: measuring the level of SYT17 protein in the urine-derived specimen collected from the test subject; comparing the level of SYT17 protein in the urine-derived specimen collected from the test subject with the level of SYT17 protein in a urine-derived specimen collected from a control subject; and, when the level of SYT17 protein in the urine-derived specimen collected from the test subject is higher than the level of SYT17 protein in the urine-derived specimen collected from the control subject, determining that the test subject is developing or has a high possibility of developing chronic kidney allograft rejection or chronic kidney disease.
(4) The method according to (1), comprising the steps of: measuring the level of SYT17 protein in a first urine-derived specimen collected from the test subject; comparing the level of SYT17 protein in the first urine-derived specimen with the level of SYT17 protein in a second urine-derived specimen collected from the same test subject prior to the collection of the first urine-derived specimen; and, when the level of SYT17 protein in the first urine-derived specimen is higher than the level of SYT17 protein in the second urine-derived specimen, determining that the test subject has a high possibility of being at a more advanced stage of the chronic kidney allograft rejection or the chronic kidney disease as of the collection of the first urine-derived specimen than as of the collection of the second urine-derived specimen.
(5) The method according to any one of (1) to (4), wherein the urine-derived specimen is a specimen enriched with urine exosome.
(6) The method according to any one of (1) to (5), wherein the test subject is a kidney transplant recipient.
(7) The method according to any one of (1) to (6), wherein a kidney disease to cause the chronic kidney disease is membranous nephropathy, nephrotic syndrome, lupus nephritis, diabetic nephropathy, IgA nephropathy, focal segmental glomerulosclerosis, autosomal dominant polycystic kidney disease, or chronic allograft nephropathy.
(8) A pharmaceutical composition for preventing and/or treating chronic kidney allograft rejection or chronic kidney disease, the pharmaceutical composition including at least one substance selected from the group consisting of a specific antibody to SYT17, a derivative of the specific antibody to SYT17, and an inhibitory nucleic acid against SYT17.
(9) The pharmaceutical composition according to (8), for administration to a subject having a urinary SYT17 protein level that is higher than a urinary SYT17 protein level in a control subject or exceeds a cutoff value, the cutoff value being determined by measuring beforehand the levels of SYT17 protein in urine-derived specimens collected from a plurality of patients developing chronic kidney allograft rejection or chronic kidney disease and a plurality of control subjects, and conducting an ROC analysis using these measured levels.
(10) The pharmaceutical composition according to (8) or (9), wherein a kidney disease to cause the chronic kidney disease is membranous nephropathy, nephrotic syndrome, lupus nephritis, diabetic nephropathy, IgA nephropathy, focal segmental glomerulosclerosis, autosomal dominant polycystic kidney disease, or chronic allograft nephropathy.
(11) A test kit for use in assessing the possibility of onset or progression of chronic kidney allograft rejection or chronic kidney disease, the test kit including a specific antibody to SYT17 or a derivative of the specific antibody to SYT17.
(12) The test kit according to (11), further including a specific antibody to CD9 or a derivative of the specific antibody to CD9.
(13) The test kit according to (11) or (12), the test kit being for use in a companion diagnosis for the pharmaceutical composition according to any one of (8) to (10).

### EFFECT OF INVENTION

According to the present invention, by using a urine-derived specimen, which can be noninvasively and easily collected, the possibility of onset or progression of chronic kidney allograft rejection or chronic kidney disease can be determined, and furthermore, chronic kidney allograft rejection and chronic kidney disease can be prevented and/or treated.

### BRIEF DESCRIPTION OF DRAWINGS

FIG. 1A is a set of typical fluorescence microscope images of immunohistochemical staining for SYT17 protein in renal graft specimens from kidney transplant recipients classified into a normal histology group (NED), an interstitial fibrosis and tubular atrophy group (IF/TA), a calcineurin inhibitors toxicity group (CNI-T), and chronic active antibody-mediated rejection group (CAAMR). FIG. 1B is a graph illustrating quantification results of the fluorescence intensity of SYT17 signals detected in immunohistochemistry by ImageJ. ^{∗∗∗}: p < 0.001 (by Tukey's test), n = 6.
FIG. 2 is a set of typical fluorescence microscope images of immunohistochemical staining for phosphorylated NF-κB p65 (p-p65) and phosphorylated STAT3 (pSTAT3) in renal graft specimens from kidney transplant recipients classified into NED, IF/TA, CNI-T, and CAAMR. In the figure, upper three rows illustrate renal tubules, and lower three rows illustrate glomeruli. Arrows in the figure indicate p-p65 and pSTAT3 double-positive cells. The scale bar represents 100 µm.
FIG. 3 is a set of graphs illustrating comparison of expression levels of IL-6, CCL20, and SYT17 in renal tubular epithelial cells stimulated with either IL-6 or TNFα or costimulated with IL-6 and TNFα. The vertical axes of the graphs represent the expression levels of each gene relative to the expression levels in unstimulated cells.
FIG. 4 is a set of graphs illustrating mRNA expression levels of IL-6 and CCL2 in human renal glomerular endothelial cells (HRGEC) and human renal proximal tubule epithelial cells (RPTEC) introduced with SYT17 knockdown siRNA (S1 to S3) and stimulated with IL-6 + TNFα or IL-6 + IL-17. In the figure, C represents control cells introduced with negative control siRNA, and P represents control cells introduced with positive control siRNA. ^{∗}: p < 0.05 (by Dunnett's test)
FIG. 5 is a set of graphs illustrating NF-κB promoter activity and IL-6 promoter activity in HEK293T cells forcibly expressing SYT17 and stimulated with TNFα. Mock represents control cells not forcibly expressing SYT17. ^{∗}: p < 0.05 (by Student's t-test)
FIG. 6A is an image of immunoblotting for SYT17 protein using whole urine specimens from healthy volunteers (Healthy), NED, and CAAMR. In the figure, PC represents a lysate of cells excessively expressing SYT17 protein. FIG. 6B is an image of immunoblotting for SYT17 protein using urine exosome fractions from the healthy volunteers, NED, and CAAMR. FIG. 6C is a graph illustrating quantification results of SYT17 and CD9 bands detected in the immunoblotting of the urine exosome fractions by using Image J software for every lane, expressed as SYT17/CD9.
FIGS. 7A to 7D are graphs of SYT17/CD9 (FIG. 7A) and existing clinical markers (N-acetyl-β-D-glucosaminidase (NAG)/Cre (FIG. 7B), urinary protein (U-TP)/Cre (FIG. 7C), and eGFR (FIG. 7D)) in urine exosome fractions from the kidney transplant recipient groups (NED, IF/TA, CNI-T, CAAMR) and healthy volunteers. ^{∗}: p < 0.05, ^{∗∗}: p < 0.01, ^{∗∗∗}: p < 0.001 (by Tukey's test).
FIGS. 8A to 8C are graphs illustrating a correlation between SYT17/CD9 and existing clinical markers (NAG/Cre (FIG. 8A), U-TP/Cre (FIG. 8B), and eGFR (FIG. 8C)), respectively.
FIG. 9 illustrates an ROC curve with SYT17/CD9 values as explanatory variables and the presence or absence of chronic rejection as response variables.
FIG. 10 is a graph illustrating SYT17/CD9 in urine exosome fractions from healthy volunteers and a chronic kidney disease patient group (CKD). ^{∗}: p < 0.05 (by Student's t-test).
FIG. 11 is an image of immunoblotting for SYT17 protein using urine exosome fractions from CKD patients. In the figure, PC represents a positive control, NC represents a negative control, and 1 to 10 represent urine exosome fractions from each of the CKD patients.
FIG. 12 is a graph illustrating SYT17/CD9 of the urine exosome fractions from the CKD patients. In the figure, the average value of the healthy volunteers is indicated with a dotted line.
FIG. 13 is a graph illustrating SYT17/CD9 of urine exosome fractions from the CKD patients. In the figure, the average value of the healthy volunteers is indicated with a dotted line.

### DESCRIPTION OF EMBODIMENTS

The present inventors confirmed that, as shown in the Examples below, little or no SYT17 protein was detected in urine exosome fractions from healthy individuals and NED, meanwhile SYT17 protein was detected from individuals developing chronic rejection after kidney transplantation at approximately 10 times higher than that from the healthy individuals or NED.

Furthermore, it was experimentally confirmed that the expression of SYT17 protein was related to the inflammation amplifier, which synergistically increases the expression and production of inflammatory mediators such as IL-6 through simultaneous activation of NF-κB and STAT3 pathways (Ogura et al., Immunity., 2008, 29 (4) 628-36.; Murakami et al., J. Exp. Med., 2011, 208(1), 103-14.; Murakami et al., Cell Reports, 2013, 3(3), 946-59).

The inflammation amplifier is a molecular mechanism that induces chronic inflammation conditions, as reported by the present inventors. When an inflammatory stimulus is applied to type I collagen-positive non-immune cells such as fibroblasts, keratinocytes, and vascular endothelial cells, NF-κB and STAT3 pathways are simultaneously activated in the non-immune cells by cytokines, such as IL-6 and IL-17, produced from the non-immune cells themselves and the induced T cells, so that the production of cytokines such as IL-6, chemokines, growth factors, and the likes are synergistically increased. The produced IL-6 acts on the non-immune cells themselves to activate the inflammation amplifier, meanwhile chemokines and growth factors cause cell infiltration to dysregulate homeostasis. The fact that the continuous activation of the inflammation amplifier induces chronic inflammatory conditions has been experimentally proved.

Specifically for SYT17 protein, as shown in the Examples below, it was found that phosphorylation (activation) of NF-κB p65 and STAT3 was enhanced in renal tubules of CAAMR; co-stimulation of human renal tubular epithelial cells with IL-6 and TNFα increased the expression levels of IL-6 and CCL20 as well as and the expression level of SYT17; knockdown of SYT17 in human renal tubular epithelial cells suppressed the expression of IL-6 and CCL2; and, forced expression of SYT17 enhanced NF-κB promoter activity and IL-6 promoter activity.

These findings indicate that SYT17 induced to express in renal tubular epithelial cells promotes inflammation via positive feedback by the inflammation amplifier. Hence, the level of SYT17 protein in a urine-derived specimen can be an effective noninvasive diagnosis marker for the onset and progression of chronic rejection and chronic kidney disease in which chronic inflammation is involved. Furthermore, it is considered that a substance capable of suppressing the expression of SYT17 and a substance capable of inhibiting the activity of SYT17 can suppress the activation of inflammation amplifier in chronic rejection and chronic kidney disease, and are therefore useful for preventing and/or treating chronic rejection or chronic kidney disease.

### Method For Assessment

A first aspect of the present invention relates to a method for assessing the possibility of the onset or progression of chronic kidney allograft rejection or chronic kidney disease in a test subject by using the level of SYT17 protein in a urine-derived specimen collected from the test subject as an indicator. Hereinafter, the present aspect will be described by illustrating preferable embodiments.

One preferable embodiment of the present aspect includes the steps of: measuring the level of SYT17 protein in a urine-derived specimen collected from a test subject; comparing the level of SYT17 protein in the urine-derived specimen collected from the test subject with a cutoff value; and, when the level of SYT17 protein in the urine-derived specimen collected from the test subject exceeds the cutoff value, determining that the test subject is developing or has a high possibility of developing chronic kidney allograft rejection or chronic kidney disease.

The urine-derived specimen in the present invention, that is, a specimen derived from urine, may be urine itself or may be a processed product of urine, such a concentrated or partially purified product of urine, and can be appropriately selected in accordance with a method for measuring SYT17 protein. For example, in the case where a highly sensitive measurement method such as TOF-MS is employed, the urine-derived specimen may be urine itself. In contrast, in the case where a general measurement method such as immunoassay is employed, the urine-derived specimen is preferably a processed product of urine from the viewpoint of enrichment of SYT17 protein.

The processed product of urine is preferably a processed product enriched with urine exosomes, and typical examples of such product include exosome fractions harvested from urine. The harvest of exosome fractions from urine can be performed by a known method using ultracentrifugation, such as a pellet-down method, a sucrose cushion method, or density gradient centrifugation. Alternatively, the harvest can be performed using, for example, MagCapture Exosome Isolation Kit PS (FUJIFILM Wako Pure Chemical Corporation), DiagExo (registered trademark) Urinary Exosome Isolation kit (101Bio, LLC), or other commercially available urinary exosome isolation kits.

The level of SYT17 protein in a urine-derived specimen can be measured by a common method capable of measuring a protein of interest in a biological sample, for example, an immunoassay targeted for SYT17 protein, or high sensitivity mass spectrometry such as TOF-MS.

The immunoassay can be performed using a specific antibody to SYT17 protein or a derivative thereof by a method known or well-known to those skilled in the art, for example, with reference to descriptions in The Immunoassay Handbook: Theory and Applications of Ligand Binding, ELISA and Related Techniques (4TH), Elsevier.

In the present invention, the specific antibody to SYT17 protein, that is, an antibody that binds specifically to SYT17 protein is an antibody that binds more preferentially to SYT17 protein than untargeted proteins. The specific antibody to SYT17 protein may also be referred to as an antibody having a high binding affinity to SYT17 protein, and is capable of binding to SYT17 protein, with an affinity at least twice, preferably at least 10 times, more preferably at least 20 times, still more preferably at least 100 times as high as an affinity to untargeted proteins. In the case where an antibody is capable of detecting the presence of SYT17 protein, without causing undesirable results, such as false positive or false negative results typically, the antibody can be considered to be a specific antibody to SYT17 protein.

The specific antibody to SYT17 protein can originate from any species including mouse, rat, shark, rabbit, pig, hamster, camel, llama, goat, and human, for example. Furthermore, the specific antibody can belong to any class of immunoglobulin molecules (for example, IgG, IgE, IgM, IgD, or IgA) and any subclass thereof, but is preferably IgG.

In the present invention, the specific antibody to be used for measuring the level of SYT17 protein may be a polyclonal antibody or a monoclonal antibody, and the specific antibody to be used in a pharmaceutical composition described later is preferably a monoclonal antibody. Alternatively, the specific antibody can be a chimeric antibody, a humanized antibody, or a human antibody.

A derivative of the specific antibody to SYT17 protein can be an antigen-binding fragment derived from the above-mentioned antibody and defined as a partial fragment of the antibody that retains the ability to bind specifically to the antigen, such as, but not limited to, Fab (fragment of antigen binding), Fab', F(ab')2, a single chain antibody (single chain Fv), a disulfide stabilized antibody (disulfide stabilized Fv), and a peptide including CDR.

The specific antibody to SYT17 protein and a derivative thereof can be produced using a method known to those skilled in the art. For example, based on the amino acid sequence of human SYT17 protein (registered with NCBI as accession number NP_057608.2, NP_001317438.1, NP_001295086.1) or the nucleotide sequence of DNA encoding human SYT17 protein (registered with NCBI as accession number NM_016524.4, NM_001308157.2, NM_001330509.1), SYT17 protein can be produced with gene recombinant techniques, and the SYT17 protein can be used as an antigen to immunize a suitable animal and furthermore fuse a B cell derived from the animal to a myeloma cell to obtain a hybridoma. For the hybridoma method, see Meyaard et al. (1997) Immunity 7:283-290; Wright et al. (2000) Immunity 13:233-242; Kaithamana et al. (1999) J.Immunol. 163:5157-5164, for example. In addition, commercially available anti-SYT17 antibodies, such as Anti-SYT17 HPA040811 (Atlas antibodies), an anti-SYT17 antibody (Proteintech), and an antibody to SYT17 (invitrogen), as well as antibodies including, as CDR sequences, amino acid sequences being the same as the CDR sequences of the above-mentioned antibodies or having 90% or higher sequence identity with the CDR sequences of the above-mentioned antibodies, and antibodies including, as heavy-chain and light-chain variable regions, amino acid sequences being the same as amino acid sequences in heavy-chain and light-chain variable regions of the above-mentioned antibodies or having 90% or higher sequence identity with the amino acid sequences, can be used.

The specific antibody to SYT17 protein and a derivative thereof may be labeled with a suitable labeling compound. Examples of the labeling compound include fluorescent substances (for example, FITC and rhodamine), metal particles such as gold colloid, fluorescent microbeads such as Luminex (registered trademark, Luminex Corporation), pigment proteins (for example, phycoerythrin and phycocyanin), radioisotopes (for example, ³H, ¹⁴C, ³²P, ³⁵S, ¹²⁵I, and ¹³¹I), enzymes (for example, peroxidase and alkaline phosphatase), biotin, and streptavidin.

The level of SYT17 protein may be expressed as the mass or amount of SYT17 protein per unit volume of urine, that is, a SYT17 protein concentration in urine. For example, in the case where employing a method capable of detecting the presence of a protein of interest as an output signal, such as fluorescence intensity in a fluorescence immunoassay, an integrated value of the output signal indicating SYT17 protein may be used as a protein level.

The level of SYT17 protein can be expressed as an absolute value or a relative value to an internal standard, depending on the measurement method. In a preferable embodiment using a specimen enriched with urine exosomes, the level of SYT17 protein can be expressed as a relative value to the level of a protein recognized as an exosome marker by those skilled in the art, such as CD9, CD63, CD81, or ALIX.

The level of SYT17 protein in a urine-derived specimen collected from a test subject is compared with a cutoff value. The cutoff value can be determined by measuring beforehand the levels of SYT17 protein in urine-derived specimens collected from a plurality of patients developing chronic kidney allograft rejection or chronic kidney disease and a plurality of control subjects, and then conducting an ROC analysis using these measured levels.

Specifically, an ROC analysis is performed using values of the levels of SYT17 protein in urine-derived specimens as explanatory variables, and the presence or absence of the onset of chronic rejection or chronic kidney disease, that is, whether the subject is a patient developing chronic kidney allograft rejection or chronic kidney disease or a control subject as response variables. Then, an ROC curve with 1-specificity (false positive rate) on the horizontal axis and sensitivity (positive rate) on the vertical axis is created. Subsequently, the cutoff value can be determined using a method commonly used for calculating a cutoff value from an ROC curve, for example, a method of setting a cutoff value to a point at which Youden's index (sensitivity + specificity-1) is at the maximum, or a method of setting a cutoff value to a point at which the distance away from the upper left corner of an ROC curve is at the minimum.

The control subject refers to a subject that is clinically determined to be developing neither chronic rejection nor chronic kidney disease. The control subject is not limited to a subject who does not exhibit any disease or symptom, other than chronic rejection and chronic kidney disease. Examples of such control subject include healthy subjects not having any special finding on kidney and others, and subjects having undergone kidney transplantation and not having any special finding on their renal graft (No Evidence of Diseases, NED).

Throughout the entirety of the present specification, the terms "test subject", "control subject", "patient", and "individual" are not limited to human, and include mammals such as rodents including mice, rats, hamsters, and guinea pigs, primates including human, chimpanzees, and rhesus monkeys, domestic animals including swine, cows, goats, horses, and sheep, and companion animals including dogs and cats. The terms "test subject", "control subject", and "patient" can be replaced with "test individual", "control individual", and "affected individual", respectively. In the present invention, the test subject, the control subject, the patient, and the individual are preferably human. Furthermore, the test subject, the control subject, and the patient preferably are animals of the same species.

The urine-derived specimen collected from the control subject is preferably prepared in the same way as the urine-derived specimen collected from the test subject. Specifically, in the case where the urine-derived specimen collected from the test subject is urine itself, urine itself is preferably used as the urine-derived specimen collected from the control subject, and in the case where the urine-derived specimen collected from the test subject is a processed product of urine, a corresponding processed product of urine is preferably used as the urine-derived specimen collected from the control subject. The level of SYT17 protein in the urine-derived specimen collected from the control subject is preferably measured by the same way as for the measurement of the level of SYT17 protein in the urine-derived specimen collected from the test subject.

In one certain embodiment, when the level of SYT17 protein is expressed as a relative value to the level of CD9 protein, the cutoff value may be 0.42 or larger. For example, when the cutoff value is set to 0.42, the onset or a high possibility of the onset of chronic kidney allograft rejection in a test subject can be assessed at a sensitivity of 77% and a specificity of 87%, as shown in the Examples below.

In the case where the level of SYT17 protein in the urine-derived specimen collected from the test subject exceeds the cutoff value, it is estimated that the test subject is developing or has a high possibility of developing chronic kidney allograft rejection or chronic kidney disease, and a physician can make a diagnosis, based on this information, and can perform prophylactic or therapeutic intervention against the onset or progression of the chronic kidney allograft rejection or the chronic kidney disease.

The test subject in the present invention is a subject that may develop chronic kidney allograft rejection or chronic kidney disease. In particular, a test subject for the assessment of the onset possibility of chronic kidney allograft rejection is a subject having undergone kidney transplantation surgery, that is, a kidney transplant recipient. The test subject for the assessment of the onset possibility of chronic kidney disease is not particularly limited, and is beneficially a subject desired to be assessed, preferably a subject suffering from a kidney disease to cause chronic kidney disease or a subject having a possibility of suffering from the kidney disease.

The kidney disease to cause the chronic kidney disease in the present invention may be a primary kidney disease, a secondary kidney disease, or a hereditary or congenital kidney disease. The kidney disease may be a glomerular disease, a vascular disease, or a tubulointerstitial disease. Examples of the kidney disease to cause the chronic kidney disease include membranous nephropathy (including idiopathic membranous nephropathy), nephrotic syndrome (including primary nephrotic syndrome and minimal change nephrotic syndrome), lupus nephritis, chronic allograft nephropathy, diabetic nephropathy, IgA nephropathy, focal segmental glomerulosclerosis, nephrosclerosis, Goodpasture's syndrome, polycystic kidney disease (including autosomal dominant polycystic kidney disease), urate nephropathy, and obesity-related nephropathy.

Another preferable embodiment of the method for assessment according to the first aspect includes the steps of: measuring the level of SYT17 protein in a urine-derived specimen collected from a test subject; comparing the level of SYT17 protein in the urine-derived specimen collected from the test subject with the level of SYT17 protein in a urine-derived specimen collected from a control subject; and, when the level of SYT17 protein in the urine-derived specimen collected from the test subject is higher than the level of SYT17 protein in the urine-derived specimen collected from the control subject, determining that the test subject is developing or has a high possibility of developing chronic kidney allograft rejection or chronic kidney disease. Descriptions of the steps and definitions of the terms are as described above unless otherwise described below.

In this embodiment, the level of SYT17 protein in the urine-derived specimen collected from the test subject is compared with the level of SYT17 protein in the urine-derived specimen collected from the control subject. The level of SYT17 protein in the urine-derived specimen collected from the control subject is preferably measured using a urine-derived specimen collected from the control subject and prepared in the same way as the urine-derived specimen collected from the test subject, by the same method as that for the measurement of SYT17 protein in the urine-derived specimen collected from the test subject. The level of SYT17 protein in the urine-derived specimen collected from the test subject and the level of SYT17 protein in the urine-derived specimen collected from the control subject do not need to be simultaneously measured, and the level of SYT17 protein in the urine-derived specimen collected from the control subject may be measured beforehand. For example, the levels of SYT17 protein in the urine-derived specimens collected from a plurality of control subjects are measured beforehand, and, from those measured levels, the average value, the median value, and other reference values are determined, and may be used for comparison with the level of SYT17 protein in the urine-derived specimen collected from the test subject.

In the case where the level of SYT17 protein in the urine-derived specimen collected from the test subject is higher than the level of SYT17 protein in the urine-derived specimen collected from the control subject, it is estimated that the test subject is developing or has a high possibility of developing chronic kidney allograft rejection or chronic kidney disease, and a physician can make a diagnosis, based on this information, and can perform prophylactic or therapeutic intervention against the onset or progression of the chronic kidney allograft rejection or the chronic kidney disease. For example, when the level of SYT17 protein in the urine-derived specimen collected from the test subject is at least 4 times, preferably at least 6 times, more preferably at least 8 times, and still more preferably at least 10 times as high as the level of SYT17 protein in the urine-derived specimen collected from the control subject, it can be determined that the test subject is developing or has a high possibility of developing chronic kidney allograft rejection. Furthermore, when the level of SYT17 protein in the urine-derived specimen collected from the test subject is higher than the level of SYT17 protein in the urine-derived specimen collected from the control subject, for example, when the level of SYT17 protein in the urine-derived specimen collected from the test subject is at least 1.2 times, preferably at least 1.5 times, more preferably at least 1.8 times, still more preferably at least twice, still further more preferably at least 3 times, and particularly preferably at least 5 times as high as the level of SYT17 protein in the urine-derived specimen collected from the control subject, it can be determined that the test subject is developing or has a high possibility of developing chronic kidney disease.

Still another preferable embodiment of the method for assessment according to the first aspect includes the steps of: measuring the level of SYT17 protein in a first urine-derived specimen collected from a test subject; comparing the level of SYT17 protein in the first urine-derived specimen with the level of SYT17 protein in a second urine-derived specimen collected from the same test subject prior to the collection of the first urine-derived specimen; and, when the level of SYT17 protein in the first urine-derived specimen is higher than the level of SYT17 protein in the second urine-derived specimen, determining that the test subject has a high possibility of being at a more advanced stage of chronic kidney allograft rejection or chronic kidney disease as of the collection of the first urine-derived specimen than as of the collection of the second urine-derived specimen. Descriptions of the steps and definitions of the terms are as described above unless otherwise described below.

In this embodiment, the level of SYT17 protein in the first urine-derived specimen collected from the test subject is compared with the level of SYT17 protein in the second urine-derived specimen. Here, the second urine-derived specimen is a urine-derived specimen collected from the same test subject prior to the collection of the first urine-derived specimen, that is, a specimen derived from urine collected from the same test subject prior to urine collection for obtaining the first urine-derived specimen. The first and second urine-derived specimens are preferably prepared in the same way, and furthermore, the level of SYT17 protein in the first urine-derived specimen and the level of SYT17 protein in the second urine-derived specimen are preferably measured using the same measurement method. The level of SYT17 protein in the first urine-derived specimen and the level of SYT17 protein in the second urine-derived specimen do not need to be simultaneously measured, and the level of SYT17 protein in the second urine-derived specimen may be measured beforehand. The terms "first" and "second" do not indicate a time series of the collection of urine-derived specimens and are used for distinguishing the urine-derived specimen to be assessed from the urine-derived specimen to be used for comparison.

In the case where the level of SYT17 protein in the first urine-derived specimen is higher than the level of SYT17 protein in the second urine-derived specimen, it is estimated that the test subject has a high possibility of being at a more advanced stage of chronic kidney allograft rejection or chronic kidney disease at a point in time when the first urine-derived specimen is collected than at a point in time when the second urine-derived specimen is collected, and a physician can make a diagnosis, based on this information and can perform prophylactic or therapeutic intervention against the onset or progression of the chronic kidney allograft rejection or the chronic kidney disease.

The method for assessment according to the first aspect may be referred to as a method for testing or determining the possibility of the onset or progression of chronic kidney allograft rejection or chronic kidney disease in the test subject by using the level of SYT17 protein in the urine-derived specimen collected from the test subject as an indicator, or may also be referred to as a method for assisting a diagnosis of the onset or progression of chronic kidney allograft rejection or chronic kidney disease in the test subject or a method of acquiring data for the diagnosis.

In addition, by monitoring the level of SYT17 protein in a urine-derived specimen from a kidney transplant recipient or a patient with kidney disease that is under intervention such as medication, it can be determined whether the intervention is prophylactically effective against the onset of chronic kidney allograft rejection or chronic kidney disease in the patient. Furthermore, by monitoring the level of SYT17 protein in a urine-derived specimen in a patient that is developing chronic kidney allograft rejection or chronic kidney disease and is under intervention such as medication, it can be determined whether the intervention is therapeutically effective against the chronic kidney allograft rejection or the chronic kidney disease in the patient. Thus, another aspect of the present invention provides a method for determining the effectiveness of prophylactic or therapeutic intervention against the onset of chronic kidney allograft rejection or chronic kidney disease.

### Pharmaceutical Composition

Another aspect of the present invention provides a pharmaceutical composition for preventing and/or treating chronic kidney allograft rejection or chronic kidney disease, the pharmaceutical composition including at least one substance selected from the group consisting of a specific antibody to SYT17, a derivative of the specific antibody, and an inhibitory nucleic acid against SYT17.

The terms "prevention" and "prophylactic intervention" used herein encompass all types of medically acceptable prophylactic intervention intended for purposes including inhibition or suppression of the affection or onset of diseases. The terms "treatment" and "therapeutic intervention" encompass all types of medically acceptable therapeutic intervention intended for purposes including cure or temporary remission of diseases. The treatment or prevention and the prophylactic intervention or therapeutic intervention of chronic kidney disease encompass medically acceptable intervention intended for various purposes including delay or stop of progression of chronic kidney disease, regression or disappearance of lesions, and prevention of onset or recurrence.

The pharmaceutical composition according to the present aspect includes at least one substance selected from the group consisting of a specific antibody to SYT17, a derivative of the specific antibody, and an inhibitory nucleic acid against SYT17, as an active ingredient. "To include as an active ingredient" used herein means to include an effective amount of the substance. The effective amount means an amount effective for the prevention and/or treatment of a disease, and is appropriately determined, depending on usage, age of the subject, state of the disease, and other conditions.

The inhibitory nucleic acid against SYT17 may be a nucleic acid capable of suppressing the transcription of mRNA (also referred to as SYT17 mRNA) that encodes SYT17 protein from SYT17 gene, a nucleic acid capable of degrading the mRNA, or a nucleic acid capable of suppressing protein translation from the mRNA. Typical examples of the inhibitory nucleic acid against SYT17 include an antisense nucleic acid and an RNA interference-inducing nucleic acid, such as siRNA, shRNA, and miRNA, that can be designed and produced by those skilled in the art, based on a nucleotide sequence of SYT17 gene or a nucleotide sequence of mRNA encoding SYT17 protein. Commercially available examples of the inhibitory nucleic acid include human si-SYT17 s28627, s28628, and s28629 (all from Thermo Fisher).

A DNA capable of inducing the transcription of the antisense nucleic acid or the RNA interference-inducing nucleic acid by being placed under the control of an appropriate expression promoter, as well as a nucleic acid of which the stability against nuclease degradation is enhanced by modifying a part of the nucleotide sequence of the antisense nucleic acid or the RNA interference-inducing nucleic acid are functionally equivalent to the antisense nucleic acid or the RNA interference-inducing nucleic acid, and are also encompassed in the inhibitory nucleic acid that can suppress expression in the present invention. The modification for enhancing the stability against nuclease degradation include 2'O-methylation, 2'-fluorination (F), and 4'-thiolation.

A chimeric RNA in which a part of ribonucleotide of the above-described RNA is replaced with a corresponding deoxyribonucleotide or a nucleotide analog is also encompassed in the inhibitory nucleic acid that can suppress expression in the present invention. Examples of the nucleotide analog include 5-position modified uridine or cytidine such as 5-(2-amino)propyl uridine and 5-bromouridine; 8-position modified adenosine or guanosine, such as 8-bromoguanosine; deazanucleotide, such as 7-deazaadenosine; and O- or N-alkylated nucleotide, such as N6-methyladenosine. The type and the number of bases modified or replaced in the above-described inhibitory nucleic acid are not particularly limited as long as the inhibitory nucleic acid does not lose the capability of suppressing the expression of its target molecule.

The above-described inhibitory nucleic acid can be artificially synthesized using genetic recombination techniques or chemical synthesis techniques. A genetic recombination method and a method for chemical synthesis of the nucleic acid, and a method for synthesis of a non-natural base and the nucleic acid including the non-natural base can be adopted from methods well-known to those skilled in the art. The nucleic acid may also be synthesized using an apparatus, such as what is called a DNA synthesizer.

The specific antibody to SYT17 and a derivative thereof to be used in the pharmaceutical composition are an antibody and a derivative of the antibody that are capable of specifically binding to SYT17 protein to neutralize the activity of SYT17 protein. Definitions of the specific antibody to SYT17 protein and the derivative thereof and descriptions of preferable embodiments and the likes are as described above in the item, Method For Assessment.

Neutralization of the activity of SYT17 protein by the specific antibody to SYT17 and a derivative thereof can be confirmed by administering the specific antibody or the derivative to an animal model for chronic kidney allograft rejection or chronic kidney disease and assessing improvement of the symptom or delay of progression of the symptom. Neutralization of the activity of SYT17 protein can also be confirmed by adding the specific antibody or the derivative thereof to renal cells, such as renal tubular cells or glomerular endothelial cells, in which the inflammation amplifier is activated by stimulation with inflammation amplifying factors, such as IL-6 or TNFα, and assessing suppression of the increased inflammation.

The pharmaceutical composition according to the present aspect is administered to a subject developing or having the risk of developing chronic kidney allograft rejection or chronic kidney disease. In particular, a subject that the pharmaceutical composition for preventing and/or treating chronic kidney allograft rejection is administered to is a subject having undergone kidney transplantation surgery, that is, a kidney transplant recipient.

In a specific embodiment, the pharmaceutical composition is administered to a subject having a urinary SYT17 protein level that is higher than a urinary SYT17 protein level in a control subject or exceeds a cutoff value. Whether the subject that the pharmaceutical composition is administered to has a urinary SYT17 protein level that is higher than a urinary SYT17 protein level in a control subject or exceeds a cutoff value can be confirmed using the method described above in the item, Method For Assessment.

A kidney disease to cause chronic kidney disease that can be prevented and/or treated by using the pharmaceutical composition is not limited as long as the enhanced expression of SYT17 is observed therein, and the kidney disease may be a primary kidney disease, a secondary kidney disease, or a hereditary or congenital kidney disease. The kidney disease may be a glomerular disease, a vascular disease, or a tubulointerstitial disease. Examples of the kidney disease to cause the chronic kidney disease include membranous nephropathy (including idiopathic membranous nephropathy), nephrotic syndrome (including primary nephrotic syndrome and minimal change nephrotic syndrome), lupus nephritis, chronic allograft nephropathy, diabetic nephropathy, IgA nephropathy, focal segmental glomerulosclerosis, nephrosclerosis, Goodpasture's syndrome, polycystic kidney disease (including autosomal dominant polycystic kidney disease), urate nephropathy, and obesity-related nephropathy.

The pharmaceutical composition can be administered by any route as long as the effective amount of the composition can be delivered to an inflammation site in the kidney, and is preferably administered parenterally, such as intravenously, intra-arterially, intraperitoneally, subcutaneously, intramuscularly, or topically. The formulation of the pharmaceutical composition is beneficially a formulation suitable for the administration route, such as injection or drops.

The pharmaceutical composition can alleviate or ameliorate the symptom of chronic kidney allograft rejection or chronic kidney disease or prevent the onset thereof, when administered to a subject that needs treatment for the chronic kidney allograft rejection or the chronic kidney disease or has the risk of the onset. In other words, still another aspect of the present invention can also be referred to as a method for preventing and/or treating chronic kidney allograft rejection or chronic kidney disease, the method including the step of administering an effective amount of at least one substance selected from the group consisting of a specific antibody to SYT17, a derivative of the specific antibody, and an inhibitory nucleic acid against SYT17, to a subject that needs treatment for chronic kidney allograft rejection or chronic kidney disease or has the risk of the onset thereof. The effective amount of the pharmaceutical composition, the formulation including the pharmaceutical composition, aspects, administration methods, and the like in the method for prevention and/or treatment are as described above.

The present invention further provides a method for preventing or treating the onset or progression of chronic kidney allograft rejection or chronic kidney disease. This method includes the step of giving prophylactic intervention or therapeutic intervention against the onset or progression of chronic kidney allograft rejection or chronic kidney disease to a subject that has been determined, by the method for assessment according to the first aspect, to be developing or have a high possibility of developing chronic kidney allograft rejection or chronic kidney disease or to a subject that has been determined, by the method for assessment according to the first aspect, to have a high possibility of being at a more advanced stage of chronic kidney allograft rejection or chronic kidney disease at a point in time when the first urine-derived specimen is collected than at a point in time when the second urine-derived specimen is collected.

An exemplary embodiment of this method includes the steps of: measuring the level of SYT17 protein in a urine-derived specimen collected from a test subject; comparing the level of SYT17 protein in the urine-derived specimen collected from the test subject with a cutoff value, the cutoff value being determined by measuring beforehand the level of SYT17 protein in urine-derived specimens collected from a plurality of patients developing chronic kidney allograft rejection or chronic kidney disease and a plurality of control subjects, and conducting an ROC analysis using these measured levels; and giving prophylactic intervention or therapeutic intervention against the onset or progression of the chronic kidney allograft rejection or the chronic kidney disease to the test subject, when the level of SYT17 protein in the urine-derived specimen collected from the test subject exceeds the cutoff value.

Another exemplary embodiment of this method includes the steps of: measuring the level of SYT17 protein in a urine-derived specimen collected from a test subject; comparing the level of SYT17 protein in the urine-derived specimen collected from the test subject with the level of SYT17 protein in a urine-derived specimen collected from a control subject; and giving prophylactic intervention or therapeutic intervention against the onset or progression of chronic kidney allograft rejection or chronic kidney disease to the test subject, when the level of SYT17 protein in the urine-derived specimen collected from the test subject is higher than the level of SYT17 protein in the urine-derived specimen collected from the control subject.

Still another exemplary embodiment of this method includes the steps of: measuring the level of SYT17 protein in a first urine-derived specimen collected from a test subject; comparing the level of SYT17 protein in the first urine-derived specimen with the level of SYT17 protein in a second urine-derived specimen collected from the same test subject prior to the collection of the first urine-derived specimen; and giving prophylactic intervention or therapeutic intervention against the onset or progression of chronic kidney allograft rejection or chronic kidney disease to the test subject, when the level of SYT17 protein in the first urine-derived specimen is higher than the level of SYT17 protein in the second urine-derived specimen.

In the method for preventing or treating the onset or progression of chronic kidney allograft rejection or chronic kidney disease, the prophylactic intervention or the therapeutic intervention is preferably dialysis, kidney transplantation, or the administration of the above-described pharmaceutical composition.

### Test Kit

Another aspect of the present invention provides a test kit for use in assessing the possibility of the onset or progression of chronic kidney allograft rejection or chronic kidney disease, the test kit including a specific antibody to SYT17 or a derivative thereof.

In addition to the specific antibody to SYT17 or the derivative thereof, the test kit may include a specific antibody to a protein recognized as an exosome marker, such as CD9, CD63, CD81, or ALIX, or a derivative of the specific antibody, and particularly preferably may include a specific antibody to CD9 or a derivative thereof. The test kit may further include devices and reagents that are necessary for immunoassays, for example, carriers such as a plate, and reagents such as a blocking solution, a washing solution, and a chromogenic substrate.

The test kit according to the present aspect can be used for measuring the level of SYT protein in a urine-derived specimen collected from a test subject in the above-described assessment method. Furthermore, the test kit according to the present aspect can be used for selecting a subject for administration of the above-described pharmaceutical composition, specifically used for selecting a subject having a urinary SYT17 protein level that is higher than a urinary SYT17 protein level in a control subject or exceeds a cutoff value, and thus, the test kit is useful also as a companion diagnostic agent.

The present invention will be described in more detail by the Examples below, but is not limited to these examples.

### [Examples]

### Experiment Method

### 1) Patient and Urine Specimen

With the approval by the medical ethics committee of Hokkaido University Hospital, whole urines were obtained from healthy volunteers, chronic kidney disease (CKD) patients, and kidney transplantation (KTx) patients from 2016 through 2019. Urines from the KTx patients were collected when a protocol biopsy was performed. Urines from the KTx patients collected at the time of episode biopsy were excluded. The urines were centrifuged for 5 minutes at 1,500 rpm to harvest supernatants, and the harvested supernatants were stored at -80°C.

All the KTx patients had undergone standard immunosuppression treatment using prednisone, mycophenolic acid, and tacrolimus or cyclosporine. Furthermore, from histological results of the protocol biopsy, the KTx patients were classified into 4 groups, namely, a normal histology group (NED, n = 20), an interstitial fibrosis and tubular atrophy group (IF/TA, n = 19), a calcineurin inhibitors toxicity group (CNI-T, n = 17), and a chronic active antibody-mediated rejection group (CAAMR, n = 22), based on a Banff classification (2017).

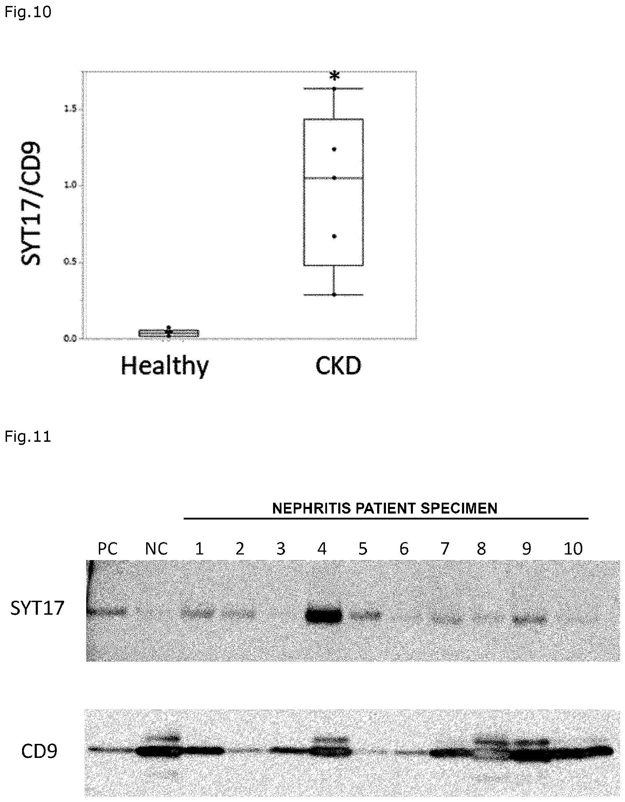

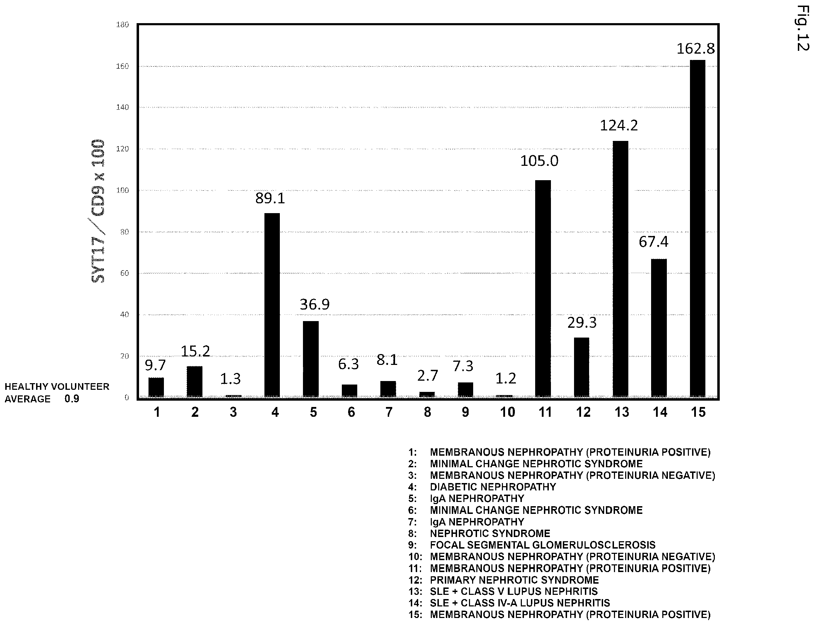

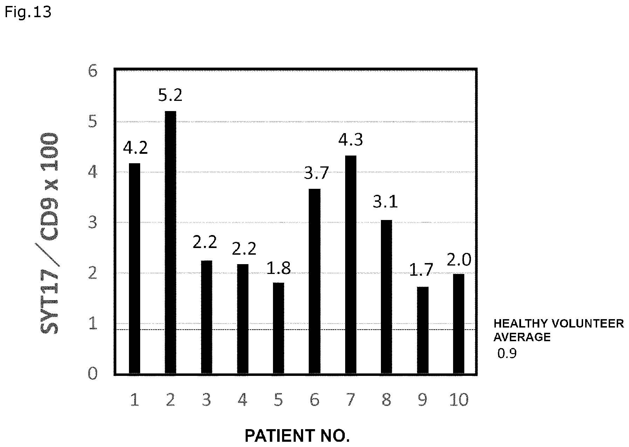

In a test with the results illustrated in FIG. 10, the CKD patients were membranous nephropathy patients (n = 2, both are proteinuria positive), a primary nephrotic syndrome patient (n = 1), and lupus nephritis patients (n = 2). In tests with the results illustrated in FIG. 11 and FIG. 12, the CKD patients were membranous nephropathy patients (proteinuria positive: n = 1, proteinuria negative: n = 2), minimal change nephrotic syndrome patients (n = 2), a nephrotic syndrome patient (n = 1), a diabetic nephropathy patient (n = 1), IgA nephropathy patients (n = 2), and a focal segmental glomerulosclerosis patient (n = 1). In a test with the results illustrated in FIG. 13, the CKD patients were autosomal dominant polycystic kidney disease patients (n = 10).

### 2) Immunohistochemical Staining

In accordance with a method described in Kawamoto, et al., 2003. Arch. Histol. Cytol. 66: 123, frozen sections or paraffin sections were prepared from surplus specimens of renal graft obtained from protocol biopsy of the KTx patients. More specifically, first, the renal graft specimens were embedded in compound and frozen at - 80°C. Frozen sections (10 µm) were prepared using a microtome, dehydrated with ethanol, and fixed with formaldehyde. For the detection of SYT17 protein, anti-SYT17 antibody (1/200, Proteintech) and rabbit IgG isotype control (1/5000, Cell Signaling Technology) were added as primary antibodies, and the resultant mixture was allowed to react at 4°C overnight. Then, donkey-anti-rabbit IgG (H+L), Alexa Fluor 546 (1/1000, Life Technologies), or donkey-anti-rabbit IgG (H+L), Hoechst 33342 trihydrochloride trihydrate (1/3,000, Life Technologies) were added as secondary antibodies and the resultant mixture was allowed to react at room temperature. Sections after the reaction were embedded in 25% glycerol and fixed with formalin to prepare paraffin sections.

Phosphorylated STAT3 and phosphorylated NF-κB were detected on serial sections using rabbit-anti-STAT3 pY705 (1:200, Cell Signaling Technology), rabbit anti-phosphorylated NF-κB p65 Ser276 (1/400, Sigma-Aldrich), or a control antibody (Cell Signaling Technology), and furthermore using a rabbit IgG Elite ABC Kit and ImmPACT DAB (Vector Laboratories, Burlingame) for signal enhancement and visualization in immunostaining. The sections were analyzed using BZ-9000 microscope (KEYENCE).

### 3) Isolation of Urinary Exosome

Using MagCapture Exosome Isolation Kit PS (FUJIFILM Wako Pure Chemical Corporation) in accordance with the manufacturer's protocol, exosomes were harvested from 1 ml of whole urine specimen, and 1/100 volume of protease inhibitor (Z-Leu-Leu-Leu-al (registered trademark), Sigma-Aldrich) was added, and then the mixture was stored at -20°C.

### 4) Immunoblotting

The whole urine specimens and the urinary exosome fractions were dissolved in SDS buffer (10% SDS, 10% glycerol, 0.04% bromophenol blue, 0.5M Tris-HCl pH 6.8, and 10% 2-mercaptoethanol), and boiled at 60°C for 10 minutes. After separation by SDS-PAGE, the proteins in the gel were transferred to PVDF membranes (Millipore). Immunoblotting was conducted using Can Get Signal Immunoreaction Enhancer Solution (Toyobo) in accordance with the manufacturer's protocol. Anti-SYT17 antibody (1/2500, HPA040811, Atlas Antibodies) and anti-CD9 antibody (1/1000, System Biosciences) were used as primary antibodies, and goat-anti-rabbit lgG(H+L) HRP antibody (1/10,000, Thermo Fisher Scientific) and goat-anti-rabbit HRP antibody (1/20000, System Biosciences) were used as secondary antibodies. For the detection of SYT17, a signal enhancing solution, Pierce (registered trademark) Western Blot Signal Enhancer (Thermo Fisher Scientific) was utilized. Protein visualization was conducted using Pierce (trademark) ECL + Western Blotting Substrate (Thermo Fisher Scientific) in accordance with the manufacturer's protocol. Films were developed using an automatic X-ray film developing device (FPM 100, Fuji Medical Film). The results of immunoblotting were quantified using Image J software.

### 5) Introduction of Expression Vector into Cells and Reporter Assay

Using polyethyleneimine, pEF-BOS vector into which SYT17 gene was incorporated (Mizushima, S. et al., 1990, Nucleic Acids Res 18: 5322) was transfected into two types of HEK293T cells that were each transformed in advance with pGL4.20 [luc2/Puro] (Promega) having firefly luciferase gene under the IL-6 promoter or pGL4.32 [luc2P/NF-κB-RE/Hygro] (Promega) having firefly luciferase gene under the NF-κB promoter, and an expression vector pGL4.74 [hRluc/TK](Promega) of Renilla luciferase serving as an internal control. Cells were harvested 24 hours after the transfection and stimulated with TNFα (50 ng/ml) for 6 hours. The cells were harvested to prepare whole cell lysates, and the changes in the promoter activity of the IL-6 promoter and the NF-κB promoter in the presence or absence of TNFα stimulation was evaluated by dual luciferase assay. The luciferase activity was measured by a luminometer (GloMax (registered trademark), Multi Detection System, Promega) using the Dual-Luciferase Reporter Assay System (Promega). The luminescence intensity from firefly luciferase reaction was corrected with the luminescence intensity from Renilla luciferase reaction.

### 6) Knockdown by siRNA

Using human si-SYT17 (S1: s28627, S2: s28628, S3: s28629, all from Thermo Fisher) as siRNA capable of knocking down the expression of SYT17, human si-nontarget (Ambion Silencer select 4390843) as a negative control, and p65 siRNA (Ambion Silencer select 4427038) capable of knocking down the expression of NF-κB p65 subunit as a positive control, the following experiment was conducted. Nucleotide sequences of the human si-SYT17 are as follows.
S1: s28627
   Sense strand sequence 5'-GCCUAGUGUUUACAGUUUUtt-3' (SEQ ID NO: 1)
   Antisense strand 5'-AAAACUGUAAACACUAGGCtg-3' (SEQ ID NO: 2)
S2: s28628
   Sense strand 5'-GCUGGUGCAUGGACUCAAAtt-3' (SEQ ID NO: 3)
   Antisense strand 5'-UUUGAGUCCAUGCACCAGCtg-3' (SEQ ID NO: 4)
S3: s28629
   Sense strand 5'-GCUCUCCACUCAUCGAUAUtt-3' (SEQ ID NO: 5)
   Antisense strand 5'-AUAUCGAUGAGUGGAGAGCtg-3' (SEQ ID NO: 6)

On Day 1, human renal proximal tubule epithelial cells (RPTEC) or human renal glomerular endothelial cells (HRGEC) were seeded at 1.4 × 10³ cells/well on 96-well plates, and siRNA solution (18 µl per well of Opti-MEM (Thermo Fisher) and 0.5 µl per well of a 5 µM siRNA solution) and transfection reagent (0.28 µl per well of Lipofectamine RNAiMAX (Thermo Fisher Scientific)) were added to each well, and cultured overnight. On Day 2, a culture medium obtained by mixing 10% FBS (Thermo Fisher Scientific) in 180 µl of DMEM (FUJIFILM Wako Pure Chemical Corporation) was added and cultured further overnight. On Day 3, after serum-starvation for 2 hours, stimulation was performed with 100 ng/mL of human IL-6 (R&D Systems) + 100 ng/mL of human soluble IL-6Rα (R&D Systems), and 50 ng/mL of human IL-17 (PeproTech) or 50 ng/mL of human TNFα (PeproTech) for 3 hours to activate STAT3 and NF-κB. After extraction of mRNA using RNA Extraction Kit (Toyobo), reverse transcription reaction was performed, and mRNA expression levels of IL-6 and CCL2 for housekeeping gene GAPDH were measured by quantitative PCR. The following primers were used.
IL-6:
   Forward primer 5'-GGTACATCCTCGACGGCATCT-3' (SEQ ID NO: 7)
   Reverse primer 5'-GTGCCTCTTTGCTGCTTTCAC-3' (SEQ ID NO: 8)
CCL2:
   Forward primer 5'-CAGCCAGATGCAATCAATGCC-3' (SEQ ID NO: 9)
   Reverse primer 5'-TGGAATCCTGAACCCACTTCT-3' (SEQ ID NO: 10)
GAPDH:
   Reverse primer 5'-GAGTCAACGGATTTGGTCGT-3' (SEQ ID NO: 11)
   Reverse primer 5'-CGCTCCTGGAAGATGGTG-3' (SEQ ID NO: 12)

### 7) Statistics Analysis

Statistics analysis was conducted with setting the sample size of each KTx group to 20. Assuming that an effect size is approximately 1.4 and a two-sided significance level is 0.05, this sample size yields 80% power when Tukey's test is employed. For comparisons between two groups, chi-square test or Student's t-test was employed. For comparisons between multiple groups, Tukey's test or Dunnett's test was employed. The ROC curve indicates the performance of binary classification (the presence of rejection vs the absence of rejection). Using Youden's index, a cutoff value was determined from the ROC curve. For the statistics analysis, JMP (registered trademark) 14 (SAS Institute Inc.) was employed. p values of less than 0.05 were considered as significant differences between groups (^{∗}P < 0.05, ^{∗∗}P < 0.01, ^{∗∗∗}P < 0.001). Error bars indicate the standard deviation (SD) of each group.

### Examples

### 1) Analysis of SYT17 Protein Expression in Renal Tissue

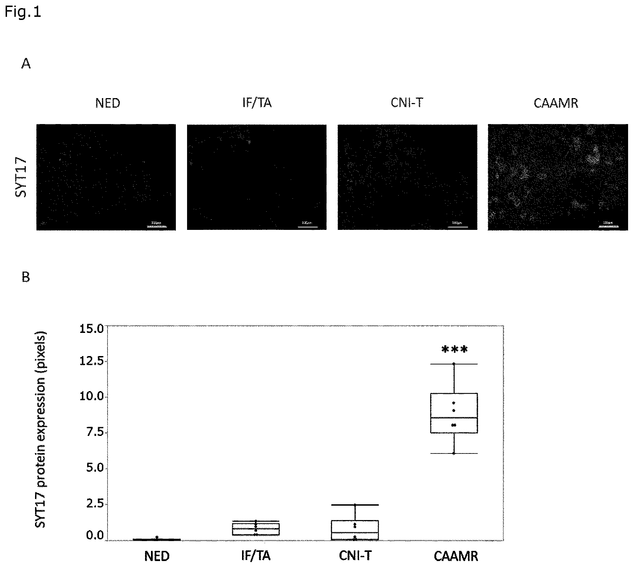

Immunohistochemical staining of renal graft specimens showed that SYT17 protein was more strongly expressed mainly in renal tubular epithelial cells in the CAAMR group, compared with the NED group, the IF/TA group, and the CNI-T group (FIG. 1). SYT17 protein was found to be localized mainly in cytoplasm (not shown).

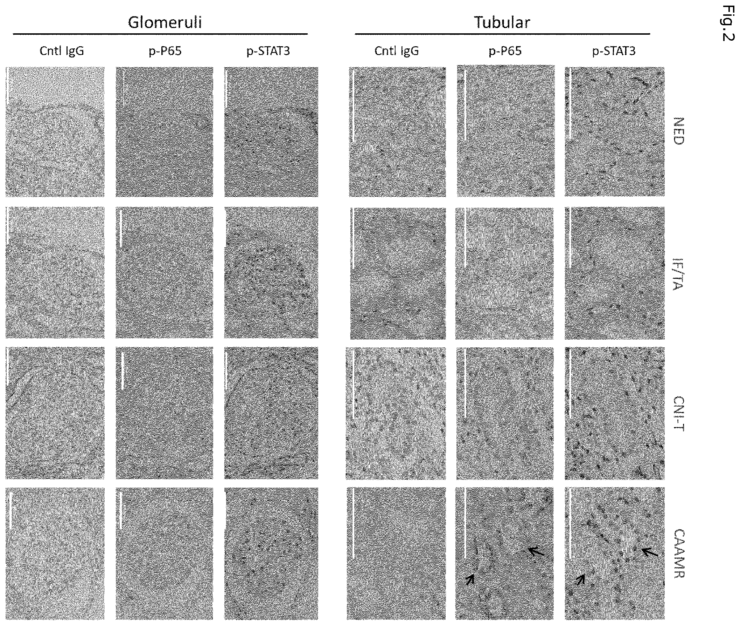

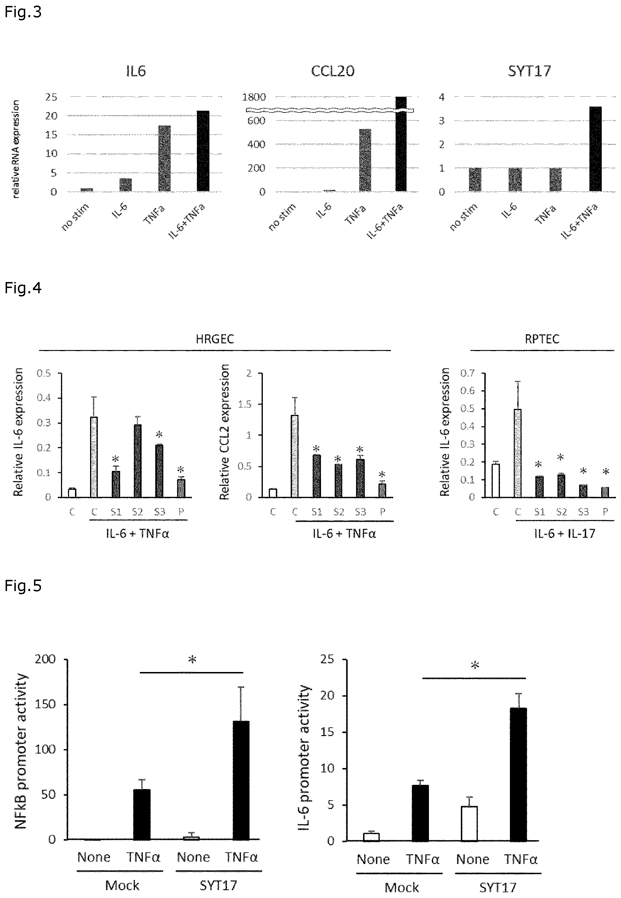

It was also confirmed that the phosphorylation (activation) of NF-κB p65 and STAT3 was enhanced in the renal tubular region of the CAAMR group, compared with the other KTx groups (FIG. 2). Furthermore, transcriptome analysis showed that co-stimulation of human renal tubular epithelial cells with IL-6 and TNFα increased the expression levels of IL-6 and CCL20 as well as the expression level of SYT17 increased (FIG. 3).

### 2) SYT17 Knockdown

Human renal glomerular endothelial cells (HRGEC) and renal proximal tubule epithelial cells (RPTEC) were transfected with each of 3 types of si-RNA, namely S1 to S3, capable of knocking down the expression of SYT17. Stimulation of both cells with IL-6 + TNFα or IL-6 + IL-17, respectively, suppressed the expression of IL-6 and CCL2 (FIG. 4).

### 3) Forced Expression of SYT17

NF-κB promoter activity and IL-6 promoter activity were evaluated by luciferase assay when HEK293T cells with forced expression of SYT17 were stimulated with TNFα. It was confirmed that NF-κB promoter activity and IL-6 promoter activity were enhanced by overexpression of SYT17 and stimulation with TNFα (FIG. 5).

These results indicate that, in renal tubular cells of CAAMR, NF-κB and STAT3 are activated by inflammation amplifying factors, such as IL-6 and CCL20, and this activation further induces the expression of SYT17 protein, and inflammation in renal graft is promoted via positive feedback.

### 4) Detection of SYT17 Protein in KTx Patient Urinary Exosome

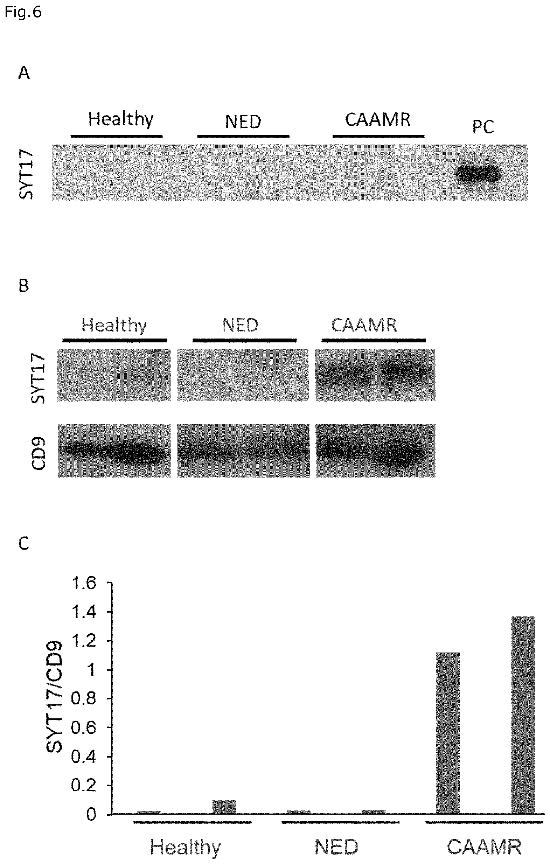

Immunoblotting of whole urine specimens and urinary exosome fractions showed that SYT17 protein was not detectable in the whole urine specimens but was detectable in the urinary exosome fractions, and that, in the CAAMR group, the relative level of SYT17 protein to an exosome marker CD9 (SYT17/CD9) was higher compared with those in the healthy volunteers and the NED group (FIG. 6).

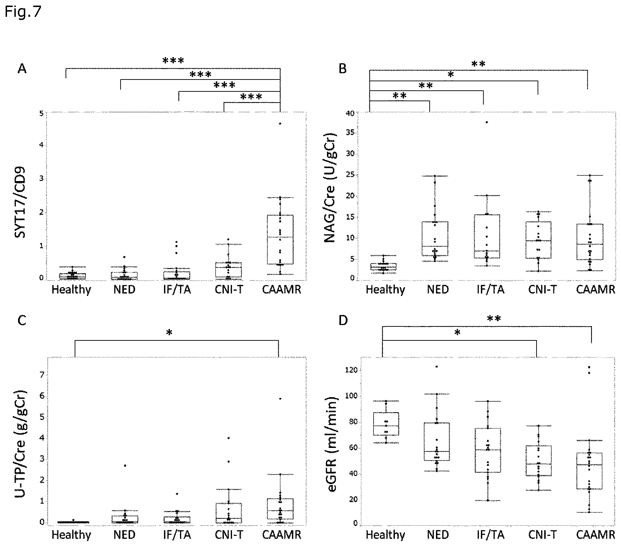

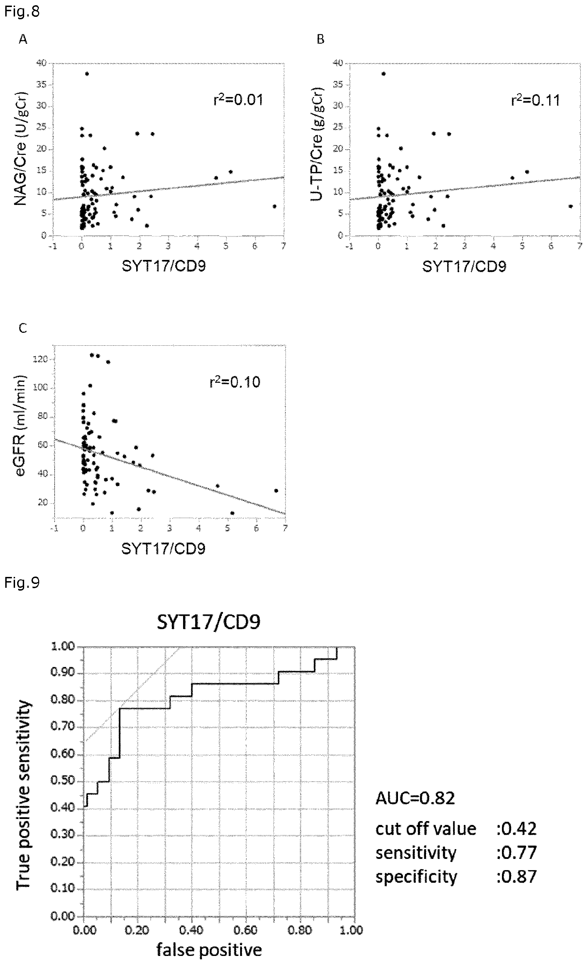

The results of SYT17/CD9 quantification in the urinary exosome fractions of a larger number of specimens, are shown together with the results of other clinical parameters, in FIGS. 7A to 7D. SYT17/CD9 was 0.12 ± 0.09 in the healthy volunteer group; 0.13 ± 0.17 in the NED group; 0.22 ± 0.35 in the IF/TA group; 0.40 ± 0.35 in the CNI-T group; and 1.34 ± 1.05 in the CAAMR group, and, in the CAAMR group, the relative SYT17 protein level in the urinary exosomes was significantly higher than those in other groups (FIG. 7A). In contrast, the currently clinically used markers, N-acetyl-β-D-glucosaminidase (NAG)/Cre, urinary protein (U-TP)/Cre, and eGFR, failed to distinguish the CAAMR group from the other groups (FIG. 7B to FIG. 7D). The significant correlation between SYT17/CD9 and those clinical parameters was not observed (FIGS. 8A to 8C). ROC analysis in which values of SYT17/CD9 were taken as explanatory variables, and the presence or absence of chronic rejection, that is, whether to belong to the CAAMR group or the other groups (the healthy volunteer group, the NED group, the IF/TA group, and the CNI-T group) was taken as response variables indicated that the ROC curve had AUC = 0.82 with moderate accuracy (FIG. 9). The optimal cutoff value for SYT17/CD9 by the ROC analysis was 0.42, with a sensitivity of 77% and specificity of 87%.

### 5) Detection of SYT17 Protein in CKD Patient Urinary Exosome

The same results as those in the CAAMR patients were confirmed in urinary exosome fractions from CKD patients (FIG. 10).

Using a larger number of specimens, SYT17 protein contained in the urinary exosome fractions from the CKD patients was detected by immunoblotting (FIG. 11 illustrates one example thereof), and the intensities of the detected SYT17 and CD9 bands were quantified for every lane to calculate SYT17/CD9 (FIG. 12 and FIG. 13). The numbers 1 to 10 in FIG. 11 and FIG. 12 correspond to the following data on CKD patient urinary exosome fractions, respectively.
1: Membranous Nephropathy (Proteinuria Positive)
2: Minimal Change Nephrotic Syndrome
3: Membranous Nephropathy (Proteinuria Negative)
4: Diabetic Nephropathy
5: IgA Nephropathy
6: Minimal Change Nephrotic Syndrome
7: IgA Nephropathy
8: Nephrotic Syndrome
9: Focal Segmental Glomerulosclerosis
10: Membranous Nephropathy (Proteinuria Negative)

The five bars (from No. 11 to No. 15) on the right side in FIG. 12 indicate quantitative values for each of the five CKD-patients analyzed in the test with the results illustrated in FIG. 10. In FIG. 12 and FIG. 13, an average value of SYT17/CD9, that is, 0.9, quantified in the same way from urinary exosome fractions from the healthy volunteers (n= 15) is indicated with a dotted line (the standard deviation of SYT17/CD9 was approximately 0.2).

In all the CKD patients, SYT17/CD9 in the urinary exosome fractions was higher than the average value of that in the healthy volunteers. In the membranous nephropathy (proteinuria negative) patients, SYT17/CD9 was larger than the average value in the healthy volunteers. In the autosomal dominant polycystic kidney disease patients, SYT17/CD9 was at least twice as high as the average value in the healthy volunteers. In the other CKD patients (membranous nephropathy patients (proteinuria positive), minimal change nephrotic syndrome patients, a diabetic nephropathy patient, IgA nephropathy patients, nephrotic syndrome patients, a focal segmental glomerulosclerosis patient, and lupus nephritis patients), SYT17/CD9 was at least 3 times as high as the average value in the healthy volunteers.

The results described in the Examples above indicate that the level of urinary SYT17 protein, in particular, the level of SYT17 protein in urinary exosome fractions serve as a diagnostic marker for chronic rejection or chronic kidney disease.

### [Sequence Listing Free Text]

SEQ ID NO: 1: Nucleotide sequence of the sense strand of human si-SYT17 s28627, siRNA against SYT17
SEQ ID NO: 2: Nucleotide sequence of the antisense strand of human si-SYT17 s28627, siRNA against SYT17
SEQ ID NO: 3: Nucleotide sequence of the sense strand of human si-SYT17 s28628, siRNA against SYT17
SEQ ID NO: 4: Nucleotide sequence of the antisense strand of human si-SYT17 s28628, siRNA against SYT17
SEQ ID NO: 5: Nucleotide sequence of the sense strand of human si-SYT17 s28629, siRNA against SYT17
SEQ ID NO: 6: Nucleotide sequence of the antisense strand of human si-SYT17 s28629, siRNA against SYT17
SEQ ID NO: 7: Nucleotide sequence of IL-6 forward primer
SEQ ID NO: 8: Nucleotide sequence of IL-6 reverse primer
SEQ ID NO: 9: Nucleotide sequence of CCL2 forward primer
SEQ ID NO: 10: Nucleotide sequence of CCL2 reverse primer
SEQ ID NO: 11: Nucleotide sequence of GAPDH forward primer
SEQ ID NO: 12: Nucleotide sequence of GAPDH reverse primer

## Claims

1. A method for assessing a possibility of onset or progression of chronic kidney allograft rejection or chronic kidney disease in a test subject by using a level of SYT17 protein in a urine-derived specimen collected from the test subject as an indicator.

2. The method according to claim 1, comprising the steps of:
measuring the level of SYT17 protein in the urine-derived specimen collected from the test subject;
comparing the level of SYT17 protein in the urine-derived specimen collected from the test subject with a cutoff value, the cutoff value being determined by measuring beforehand levels of SYT17 protein in urine-derived specimens collected from a plurality of patients developing chronic kidney allograft rejection or chronic kidney disease and a plurality of control subjects, and conducting an ROC analysis using levels obtained by the measurement; and,
when the level of SYT17 protein in the urine-derived specimen collected from the test subject exceeds the cutoff value, determining that the test subject is developing or has a high possibility of developing chronic kidney allograft rejection or chronic kidney disease.

3. The method according to claim 1, comprising the steps of:
measuring the level of SYT17 protein in the urine-derived specimen collected from the test subject;
comparing the level of SYT17 protein in the urine-derived specimen collected from the test subject with a level of SYT17 protein in a urine-derived specimen collected from a control subject; and,
when the level of SYT17 protein in the urine-derived specimen collected from the test subject is higher than the level of SYT17 protein in the urine-derived specimen collected from the control subject, determining that the test subject is developing or has a high possibility of developing chronic kidney allograft rejection or chronic kidney disease.

4. The method according to claim 1, comprising the steps of:
measuring a level of SYT17 protein in a first urine-derived specimen collected from the test subject;
comparing the level of SYT17 protein in the first urine-derived specimen with a level of SYT17 protein in a second urine-derived specimen collected from the same test subject prior to the collection of the first urine-derived specimen; and,
when the level of SYT17 protein in the first urine-derived specimen is higher than the level of SYT17 protein in the second urine-derived specimen, determining that the test subject has a high possibility of being at a more advanced stage of chronic kidney allograft rejection or chronic kidney disease as of the collection of the first urine-derived specimen than as of the collection of the second urine-derived specimen.

5. The method according to any one of claims 1 to 4, wherein the urine-derived specimen is a specimen enriched with urine exosome.

6. The method according to any one of claims 1 to 5, wherein the test subject is a kidney transplant recipient.

7. The method according to any one of claims 1 to 6, wherein a kidney disease to cause the chronic kidney disease is membranous nephropathy, nephrotic syndrome, lupus nephritis, diabetic nephropathy, IgA nephropathy, focal segmental glomerulosclerosis, autosomal dominant polycystic kidney disease, or chronic allograft nephropathy.

8. A pharmaceutical composition for preventing and/or treating chronic kidney allograft rejection or chronic kidney disease, the pharmaceutical composition comprising at least one substance selected from the group consisting of a specific antibody to SYT17, a derivative of the specific antibody, and an inhibitory nucleic acid against SYT17.

9. The pharmaceutical composition according to claim 8, for administration to a subject having a urinary SYT17 protein level that is higher than a urinary SYT17 protein level in a control subject or exceeds a cutoff value, the cutoff value being determined by measuring beforehand levels of SYT17 protein in urine-derived specimens collected from a plurality of patients developing chronic kidney allograft rejection or chronic kidney disease and a plurality of control subjects, and conducting an ROC analysis using levels obtained by the measurement.

10. The pharmaceutical composition according to claim 8 or 9, wherein a kidney disease to cause the chronic kidney disease is membranous nephropathy, nephrotic syndrome, lupus nephritis, diabetic nephropathy, IgA nephropathy, focal segmental glomerulosclerosis, autosomal dominant polycystic kidney disease, or chronic allograft nephropathy.

11. A test kit for use in assessing a possibility of onset or progression of chronic kidney allograft rejection or chronic kidney disease, the test kit comprising: a specific antibody to SYT17 or a derivative of the specific antibody to SYT17.

12. The test kit according to claim 11, further comprising: a specific antibody to CD9 or a derivative of the specific antibody to CD9.

13. The test kit according to claim 11 or 12, being for use in a companion diagnosis for the pharmaceutical composition according to any one of claims 8 to 10.
